# EUROPÄISCHE PATENTSCHRIFT

(11) **EP 1 558 641 B1**
(45) Veröffentlichungstag und Bekanntmachung des Hinweises auf die Patenterteilung: **24.02.2010**
(21) Anmeldenummer: 03757992.7
(22) Anmeldetag: 16.10.2003
(51) Int. Cl.: C07K 14/62, A61K 9/00, A61K 38/28

(54) **KRISTALLE VON INSULINANALOGA UND VERFAHREN ZU IHRER HERSTELLUNG**
CRYSTALS OF INSULIN ANALOGUES AND METHOD FOR THE PRODUCTION THEREOF
CRISTAUX D'ANALOGUES D'INSULINE ET PROCEDE DE PRODUCTION DESDITS CRISTAUX

(30) Priorität: 29.10.2002 DE 10250297
(43) Veröffentlichungstag der Anmeldung: 03.08.2005
(73) Patentinhaber: Sanofi-Aventis Deutschland GmbH, 65929 Frankfurt am Main (DE)
(72) Erfinder: BERCHTOLD, Harald, 61476 Kronberg (DE)
(86) Internationale Anmeldenummer: PCT/EP2003/011470
(87) Internationale Veröffentlichungsnummer: WO 2004/039838

(56) Entgegenhaltungen:
- EP-A- 0 709 395
- EP-A- 0 885 961
- WO-A-98/42749
- WO-A-02/076495

## Beschreibung

Die Erfindung betrifft Kristalle eines Insulinanalogons, in welchem Asparagin (Asn) in Position B3 der B-Kette durch einen natürlich auftretenden basischen Aminosäurerest ausgetauscht ist und wenigstens ein Aminosäurerest in den Positionen B27, B28 oder B29 der B-Kette durch einen anderen natürlich auftretenden neutralen oder sauren Aminosäurerest ausgetauscht ist, wobei fakultativ Phenylalanin (Phe) in Position B1 der B-Kette fehlen kann, wobei die Kristalle in der Raumgruppe R3 (Nr. 146) mit den Zellachsen A=81,5 Å ± 1 Å und C= 33,3 Å ± 1 Å vorliegen.

Weltweit leiden etwa 120 Mio. Menschen an Diabetes mellitus. Darunter sind etwa 12 Mio. Typ I-Diabetiker, für die die Applikation von Insulin die einzig derzeit mögliche Therapie darstellt. Die Betroffenen sind lebenslang, in der Regel mehrmals täglich, auf Insulininjektionen angewiesen. Obgleich Typ II-Diabetes, an dem etwa 100 Mio. Menschen leiden, nicht grundsätzlich mit einem Mangel an Insulin einhergeht, wird doch in einer Vielzahl von Fällen die Behandlung mit Insulin als günstigste oder einzig mögliche Therapieform angesehen.

Mit fortschreitender Dauer der Krankheit leidet eine große Zahl der Patienten an sogenannten diabetischen Spätkomplikationen. Es handelt sich dabei im wesentlichen um mikro- und makrovaskuläre Schädigungen, die je nach Art und Umfang Nierenversagen, Erblindung, Verlust von Extremitäten oder ein erhöhtes Risiko für Herz/Kreistauferkrankungen zur Folge haben.

Als Ursache werden in erster Linie chronisch erhöhte Blutglucosespiegel verantwortlich gemacht, da auch bei sorgfältiger Einstellung der Insulintherapie ein normales Blutglucoseprofil, wie es der physiologischen Regulation entsprechen würde, nicht erreicht wird (Ward, J. D. (1989) British Medical Bulletin 45, 111-126; Drury, P.L. et al. (1989) British Medical Bulletin 45,127-147; Kohner, E.M. (1989) British Medical Bulletin 45, 148-173).

Beim Gesunden ist die Insulinsekretion eng an die Glucosekonzentration des Blutes angelehnt. Erhöhte Glucosespiegel, wie sie nach den Mahlzeiten auftreten, werden durch eine gesteigerte Insulinfreisetzung rasch kompensiert. Im nüchternen Zustand sinkt der Plasmainsulinspiegel auf einen basalen Wert ab, der ausreicht, eine kontinuierliche Versorgung insulinsensitiver Organe und Gewebe mit Glucose zu gewährleisten. Eine Optimierung der Therapie, die sogenannte intensivierte Insulintherapie, zielt heute primär darauf ab, Schwankungen der Blutglucosekonzentration, speziell Entgleisungen nach oben, möglichst gering zu halten (Bolli, G. B. (1989) Diabetes Res. Clin. Pract. 6, S3-S16; Berger, M. (1989) Diabetes Res. Clin. Pract. 6, S25-S32). Dies führt zu einer signifikanten Verminderung des Auftretens und des Fortschreitens diabetischer Spätschäden (The Diabetes Control and Complications Trial Research Group (1993) N. Engl. J. Med. 329, 977-986).

Aus der Physiologie der Insulinsekretion läßt sich ableiten, daß für eine verbesserte, intensivierte Insulintherapie unter Verwendung subcutan applizierter Präparate zwei Insulinzubereitungen mit unterschiedlicher Pharmakodynamik benötigt werden. Zur Kompensation des Blutglucoseanstiegs nach den Mahlzeiten muß das Insulin rasch anströmen und darf nur einige Stunden wirken. Zur basalen Versorgung, insbesondere in der Nacht, sollte ein Präparat zur Verfügung stehen, das lange wirkt, kein ausgeprägtes Maximum aufweist und nur sehr langsam anströmt.

Die auf humanen und tierischen Insulinen basierenden Präparate erfüllen die Ansprüche einer intensivierten Insulintherapie jedoch nur begrenzt. Rasch wirksame Insuline (Alt-Insuline) gelangen zu langsam ins Blut und an den Wirkort und weisen eine zu lange Gesamtwirkdauer auf. Die Folge ist, daß die postprandialen Glucosespiegel zu hoch liegen und mehrere Stunden nach der Mahlzeit die Blutglucose zu stark absinkt (Kang, S. et al. (1991) Diabetes Care 14, 142-148; Home, P.J. et al. (1989) British Medical Bulletin 45, 92-110; Bolli, G. B. (1989) Diabetes Res. Clin. Pract. 6, S3-S16). Die verfügbaren Basalinsuline wiederum, vorallem NPH-Insuline, weisen eine zu kurze Wirkdauer auf und besitzen ein zu stark ausgeprägtes Maximum.

Neben der Möglichkeit, das Wirkprofil über galenische Prinzipien zu beeinflussen, bietet sich mit Hilfe der Gentechnik heute die Alternative, Insulinanaloga zu entwerfen, die bestimmte Eigenschaften wie Wirkungseintritt und -dauer allein durch ihre strukturellen Eigenschaften erzielen. Durch die Verwendung geeigneter Insulinanaloga könnte daher eine wesentlich bessere, den natürlichen Verhältnissen enger angeglichene Einstellung der Blutglucose erreicht werden.

Insulinanaloga mit beschleunigtem Wirkungseintritt werden in EP 0 214 826, EP 0 375 437 und EP 0 678 522 beschrieben. EP 0 214 826 bezieht sich u.a. auf Substitutionen von B27 und B28, jedoch nicht in Verbindung mit der Substitution von B3. EP 0 678 522 beschreibt Insulinanaloga die in der Position B29 verschiedene Aminosäuren, vorzugsweise Prolin, aufweisen, jedoch nicht Glutaminsäure. EP 0 375 437 umfaßt Insulinanaloga mit Lysin oder Arginin in B28, die optional zusätzlich in B3 und/oder A21 modifiziert sein können. In EP 0 885 961 A1 wird B3-Lysin, B29-Glutamat-Humaninsulin als neues, schnellwirkendes Insulin offenbart.

In der EP 0 419 504 werden Insulinanaloga offenbart, die gegen chemische Modifikationen geschützt sind, indem Asparagin in B3 und wenigstens eine weitere Aminosäure in den Positionen A5, A15, A18 oder A21 verändert sind. Die hier beschriebenen Insulinanaloga weisen jedoch nur eine Modifikation in der Position B3 und keine weitere aus der erwähnten Gruppe auf. Ein Hinweis, daß diese Verbindungen eine veränderte Pharmakodynamik mit der Folge eines schnelleren Wirkungseintritts besitzen, ist nicht gegeben.

Insulinanaloga sind Analoga von natürlich vorkommenden Insulinen, nämlich Humaninsulin oder tierischen Insulinen, welche sich durch Substitution wenigstens eines natürlich auftretenden Aminosäurerestes und/oder Addition wenigstens eines Aminosäurerestes und/oder organischen Restes von dem entsprechenden, ansonst gleichen natürlich vorkommenden Insulin unterscheiden.

Die A-Kette von Humaninsulin weist dabei die folgende Aminosäuresequenz auf:
Gly Ile Val Glu Gln Cys Cys Thr Ser Ile Cys Ser Leu Tyr Gln Leu Glu Asn Tyr Cys Asn (SEQ ID NO 1)

Die B-Kette von Humaninsulin weist dabei die folgende Aminosäuresequenz auf:
Phe Val Asn Gln His Leu Cys Gly Ser His Leu Val Glu Ala Leu Tyr Leu Val Cys Gly Glu Arg Gly Phe Phe Tyr Thr Pro Lys Thr (SEQ ID NO 2).

Von den zwanzig natürlich auftretenden Aminosäuren, die genetisch kodierbar sind, werden hier die Aminosäuren Glycin (Gly), Alanin (Ala), Valin (Val), Leucin (Leu), Isoleucin (Ile), Serin (Ser), Threonin (Thr), Cystein (Cys), Methionin (Met), Asparagin (Asn), Glutamin (Gln), Phenylalanin (Phe), Tyrosin (Tyr), Tryptophan (Trp) und Prolin (Pro) als neutrale Aminosäuren, die Aminosäuren Arginin (Arg), Lysin (Lys), und Histidin (His) als basische Aminosäuren und die Aminosäuren Asparaginsäure (Asp) und Glutaminsäure (Glu) als saure Aminosäuren bezeichnet.

oder dessen physiologisch verträgliches Salz, nämlich ein Insulinanalogon oder ein physiologisch verträgliches Salz der Formel I, das sich dadurch auszeichnet, daß

| | |
|---|---|
| A8 | Alanin (Ala), |
| A9 | Serin (Ser), |
| A10 | Valin (Val) und |
| B30 | Alanin (Ala) bedeuten (Aminosäurereste A8 bis A10 und B30 des Rinderinsulins), |
| A8 | Threonin (Thr), |
| A9 | Serin (Ser) und |
| A10 | Isoleucin (Ile) bedeuten (Aminosäurereste A8 bis A10 der Insuline von Mensch oder Schwein), wobei |
| B30 | Alanin (Ala) (Aminosäurerest B30 von Schweineinsulin) oder |
| B30 | Threonin (Thr) bedeutet (Aminosäurerest B30 von Humaninsulin, vgl. SEQ ID NO 2). |

Besonders bevorzugt ist ein Insulinanaloga oder ein physiologisch verträgliches Salz desselben der Formel I mit den Aminosäureresten A8 bis A10 und B30 von Humaninsulin, welches sich ferner dadurch auszeichnet, daß

| | |
|---|---|
| (A1-A5) | die Aminosäurereste in den Positionen A1 bis A5 der A-Kette von Humaninsulin (vgl. SEQ ID NO 1), |
| (A12-A19) | die Aminosäurereste in den Positionen A12 bis A19 der A-Kette von Humaninsulin (vgl. SEQ ID NO 1), |
| (B8-B18) | die Aminosäurereste in den Positionen B8 bis B18 der B-Kette von Humaninsulin (vgl. SEQ ID NO 2) und |
| (B20-B26) | die Aminosäurereste in den Positionen B20 bis B26 der B-Kette von Humaninsulin (vgl. SEQ ID NO 2) bedeuten. |

Weitere bevorzugte Ausgestaltungen der vorliegenden Erfindung sind Kristalle enthaltend ein Insulinanalogon oder ein physiologisch verträgliches Salz desselben der Formel I, dadurch gekennzeichnet, daß der Aminosäurerest in Position B1 der B-Kette ein Phenylalaninrest (Phe) ist oder
ein Insulinanalogon oder ein physiologisch verträgliches Salz desselben der Formel 1, dadurch gekennzeichnet, daß der Aminosäurerest in Position B3 der B-Kette ein Histidin- (His), Lysin- (Lys) oder Argininrest (Arg) ist.

Weitere bevorzugte Ausgestaltungen der vorliegenden Erfindung sind Kristalle enthaltend ein Insulinanalogon oder ein physiologisch verträgliches Salz desselben der Formel I , dadurch gekennzeichnet, daß wenigstens einer der Aminosäurereste in den Positionen B27, B28 und B29 der B-Kette durch einen natürlich auftretenden Aminosäurerest ersetzt ist, welcher ausgewählt ist aus der Gruppe der neutralen oder der sauren Aminosäuren,
ein Insulinanalogon oder ein physiologisch verträgliches Salz desselben der Forme I, dadurch gekennzeichnet, daß wenigstens einer der Aminosäurereste in den Positionen B27, B28 und B29 der B-Kette ein natürlich auftretender Aminosäurerest ist, welcher ausgewählt ist aus der Gruppe Isoleucin (Ile), Asparaginsäure (Asp) und Glutaminsäure (Glu), vorzugsweise dadurch gekennzeichnet, daß wenigstens einer der Aminosäurereste in den Positionen B27, B28 und B29 der B-Kette durch einen natürlich auftretenden Aminosäurerest ersetzt ist, welcher ausgewählt ist aus der Gruppe der neutralen Aminosäuren, oder besonders bevorzugt, daß wenigstens einer der Aminosäurereste in den Positionen B27, B28 und B29 der B-Kette ein Isoleucinrest (Ile) ist, oder
ein Insulinanalogon oder ein physiologisch verträgliches Salz desselben der Formel I, dadurch gekennzeichnet, daß wenigstens einer der Aminosäurereste in den Positionen B27, B28 und B29 der B-Kette ein natürlich auftretender Aminosäurerest ist, welcher ausgewählt ist aus der Gruppe der sauren Aminosäuren, vorzugsweise dadurch gekennzeichnet, daß wenigstens einer der Aminosäurereste in den Eine bevorzugte Ausgestaltung der vorliegenden Erfindung sind Kristalle enthaltend ein Insulinanalogon oder ein physiologisch verträgliches Salz desselben, das sich dadurch auszeichnet, daß die B-Kette die Sequenz

Phe Val Lys Gln His Leu Cys Gly Ser His Leu Val Glu Ala Leu
Tyr Leu Val Cys Gly Glu Arg Gly Phe Phe Tyr Thr Pro Glu Thr
aufweist (SEQ ID NO 3).

Das Insulinanalogon lässt sich vorzugsweise gentechnologisch herstellen.

Die Herstellung des Insulins erfolgt hauptsächlich gentechnologisch mittels sitedirected mutagenesis nach Standardmethoden.

Dazu wird eine für das gewünschte Insulinanalogon kodierende Genstruktur konstruiert und in einer Wirtszelle - vorzugsweise in einem Bakterium wie E. coli oder eine Hefe, insbesondere Saccharomyces cerevisiae - zur Expression gebracht und - falls die Genstruktur für ein Fusionsprotein codiert - aus dem Fusionsprotein das Insulin-Analogon freigesetzt; analoge Methoden sind z.B. beschrieben in EP-A-0 211 299, EP-A-0 227 938, EP-A-0 229 998, EP-A-0 286 956 und der DE-Patentanmeldung P 38 21 159.

Die Abspaltung des Fusionsproteinanteils kann nach Zellenaufschluß chemisch mittels Halogencyan (siehe EP-A-0 180 920) erfolgen.

Bei der Herstellung mittels eines Präproinsulinvorläufers der einen Fusionsproteinanteil (Präsequenz) nach US 5,358,857 besitzt, erfolgt die Abspaltung des Fusionsproteinanteils auf einer späteren Stufe zusammen mit der Abspaltung des C-Peptides.

Der Insulinvorläufer wird dann der oxidativen Sulfitolyse nach der z.B. von R.C. Marshall und A.S. Inglis in "Practical Protein Chemistry - A Handbook" (Herausgeber A. Darbre) 1986, Seiten 49 - 53 beschriebenen Methode unterworfen und anschließend in Gegenwart eines Thiols unter Ausbildung der korrekten Disulfidbrücken renaturiert, z.B. nach der von G.H. Dixon und A.C. Wardlow in Nature (1960), Seiten 721 - 724 beschriebenen Methode.

Die Insulinvorläufer können jedoch auch direkt gefaltet werden (EP-A-0 600 372; EP-A-0 668 292).

Das C-Peptid wird mittels tryptischer Spaltung entfernt - z.B. gemäß der Methode von Kemmler et al., J.B.C. (1971), Seiten 6786 - 6791 und das Insulinanalogon der Formel I mittels bekannter Techniken wie Chromatographie - z.B. EP-A-0 305760 - und Kristallisation gereinigt.

Bei diesen Verfahren endet die B-Kette C-terminal mit Arginin oder zwei Argininresten. Diese können enzymatisch mittels Carboxypeptidase B entfernt werden.

Das Insulinanalogon besitzt volle biologische Aktivität. Dies wurde durch intravenöse Applikation an Kaninchen und der daraus resultierenden Blutglucosesenkung gezeigt. Der schnellere Wirkungseintritt nach subcutaner Applikation wurde mit der euglykämischen Clamp Technik am nüchternen Hund gezeigt (EP 0 885 961 A1. Beispiele 5 und 6). Es wurden 0,3 IE/kg verabreicht. Referenzpräparat war Humaninsulin. Bei der Clamptechnik wird nach der Insulininjektion in kurzen Zeitabständen der Blutglucosewert gemessen und genau soviel Glucose infundiert, um die Absenkung zu kompensieren. Dies hat den Vorteil, daß bei den Tieren keine Gegenregulation auftritt, wie es bei einem starken Abfall der Blutglucose nach der Gabe von Insulin der Fall wäre. Die Menge und der zeitliche Verlauf der infundierten Glucose charakterisieren die Wirkung des Insulins. Lys(B3), Glu(B29)- (SEQ ID NO 3) weist einen deutlich schnelleren Wirkungseintritt als Humaninsulin auf. Die maximale Wirkung (Glucoseinfusionsrate) wird mit Humaninsulin nach 100 Minuten erreicht, mit Lys(B3), Glu(B29)-Insulin (SEQ ID NO 3) dagegen nach 80 Minuten. Daher sollte das genannte Analogon, wenn es kurz vor einer Mahlzeit injiziert wird, den postprandialen Anstieg der Blutglucose besser kompensieren als Humaninsulin.

Das beschriebene Insulinanalogon eignet sich sowohl zur Therapie des Typ I- als auch des Typ II-Diabetes mellitus vorzugsweise in Verbindung mit einem Basalinsulin.

Insulin-Analoga können in den pharmazeutischen Zubereitungen auch in Form deren physiologisch verträglichen Salze, als Alkali- oder als Ammoniumsalze, eingesetzt werden. Ein beliebiger Anteil eines oder mehrerer Insulin-Analoga kann in einer Mischung weiterer dieser Insulin-Analoga unabhängig voneinander jeweils in gelöster, amorpher und/oder kristalliner Form vorliegen.

Insuline und Insulinanaloga werden häufig als wässrige pharmazeutische Formulierungen bereitgestellt, die Zinkionen enthalten.

Die Zugabe von Zinkionen zu Insulinpräparationen erfolgt im wesentlichen aus zwei Gründen:
1. Zn²⁺-Ionen wirken stabilisiernd auf Insulinpräparationen da diese die Bildung von Insulin-Hexameren förderen [Brange et al. Diabetic Medicine, 3, 532-536 (1986).
2. In Abhängigkeit von der Zn²⁺ -Ionen Konzentration kann der zeitliche Verlauf der An- und Abströmkinetik von Insulinpräparationen gesteuert werden.

Bei schnellwirkenden Insulinen führen die Punkte 1 und 2 zu einer Problematik. Aufgrund der erhöhten galenischen Stabilität der Präparation sind hohe Konzentrationen an Insulin-Hexameren vorteilhaft. Da Zn²⁺-Ionen die An- und Abström-kinetik des Insulinanalogons jedoch verzögern, wirken sie entgegengesetzt zur gewünschten Kinetik der Wirkstoff-Freisetzung. Daher hat man sich entschieden, eines der beschriebenen Insulinanaloga, nämlich Lys B3, Glu B29-Humaninsulin, in Form einer zinkfreien, wässrigen Formulierung bereitzustellen (Internationale Patentanmeldung PCT/EP02/02625).

Zur Herstellung der zinkfreien, wässrigen Formulierung von Lys B3, Glu B29-Humaninsulin besteht das Bedürfnis, zinkfreie Kristalle dieses Insulinanalogons einsetzen zu können. Darüber hinaus können zinkfreie Kristalle davon auch zur Herstellung anderer pharmazeutischer Formulierungen verwendet werden, z.B. bei festen Formulierungen oder Emulsionen zur Verabreichung per Inhalator, oder bei oraler Verabreichung.

Die der Erfindung zugrundeliegende Aufgabe war somit die Bereitstellung zinkfreier Kristalle des beschriebenen Insulinanalogons. Dabei sollte auch ein Ersatz von Zinkionen durch andere zweiwertige Ionen, die für pharmazeutische Formulierungen aufgrund ihrer Toxizität ungeeignet sind, vermieden werden.

Es wurde nun überraschenderweise gefunden, dass das beschriebene Insulinanalogon in Hexamer-Kristallen hergestellt werden kann, die keine zweiwertigen Ionen wie z.B. Zinkionen, enthalten.

Diese neue Kristallform von Lys B3, Glu B29-Humaninsulin ist kristallograpisch isomorph zum klassischen 2Zn-Insulin-Typ der in der Literatur für Schweineinsulin und Humaninsulin beschrieben ist [Baker et al., Phil.Trans.Roy.Soc. 319, 369-454(1988)]. Die neuen zinkfreien Hexamer-Kristalle gehören der rhomboedrischen Raumgruppe R3 (Nr. 146) an mit zwei Insulinmonomeren pro asymmetrischer Einheit. Die Elementarzellachsen bei -70°C (durch Anwendung der Schockgefriermethode) wurden ermittelt zu: A = 81,5 Å, C = 33,3 Å. Die Röntgenstrukturanalyse der neuen Kristallform bei 1,8 Å Auflösung ergab, dass sich an Stelle der Zn²⁺-Ionen (im klassischen 2Zn-Typus) mit Bindungsabständen (Zn²⁺-His-B10) um 2,0 Å in der neuen Kristallform von Lys B3, Glu B29-Humaninsulin nur ein sehr kleiner Elektronendichtepeak befindet der mit einem Wassermolekül (H₂O) entspricht (Bindungsabstand H₂O -His-B10 um 2,3 Å). Sowohl die Quantität der Elektronendichte als auch der Bindungsabstand zeigen für ein Insulin mit Glutamat-B21-Seitenkette ein bisher unbekanntes strukturelles Arrangement der Region um die Histidin-B10-Seitenkette.

Die (1σ-2Fo-Fc)-Elektronendichte der Region um Histidin B-10 ist signifikant unterschiedlich zum klassischen 2Zn-Typus, in welchem ein Zn²⁺-Ion oktaedrisch von je drei Symmetrie-benachbarten Histindin-B10 und je drei Wassermolekülen koordiniert werden. Die Bindungsabstände (Zn²⁺-His-B10) der in der PDB-Daten-bank [Bernstein et al., J.Mol.Biol, 112, 535-542 (1977)] hinterlegten Strukturen des 2Zn-Typus liegen zwischen 1,9 und 2,1 Å und sind signifikant unterschiedlich zu den beobachteten Bindungsabstände um 2,3Å in der neuen, zinkfreien Kristallstruktur. Das besondere an der hier beschriebenen neuen Hexamer-Kristallform von Lys B3, Glu B29-Humaninsulin ist, dass diese Insulin-Hexamere enthält ohne die ansonsten hierfür notwendigen zweiwertigen Kationen (z.B. Zn²⁺, Co²⁺, Cd²⁺). Bisher hat man angenommen, dass die Ausbildung von Insulin-Hexameren ohne zweiwertige Kationen nur dann möglich ist wenn die abstoßenden Kräfte der Glutamat-B21 Seitenkette durch Protein-Engineering (z.B. durch den Austausch von B21-Glu zu Gln) reduziert werden kann [Bentley et al., J.Mol.Biol. 228,1163-1176 (1992). Die Beibehaltung der Glu-B21-Seitenkette - welche den Zerfall von Insulin-Hexameren fördert - ist jedoch vor allem für schnellwirkende Insuline zum Erhalt einer schnellen An- und Abströmkinetik wichtig.

Gegenstand der Erfindung sind demgemäß Kristalle des Insulinanalogons Lys B3, Glu B29 Humaninsulin, die Kristalle in der Raumgruppe R3 (Nr. 146) mit den Zellachsen A=81,5 Å ± 1 Å und C= 33,3 Å ± 1 Å vorliegen und wobei die Moleküle des Insulinanalogons in Form von zinkfreien Hexameren, bestehend aus jeweils drei Dimeren, vorliegen.

Ein weiterer Gegenstand der Erfindung sind Kristalle wie oben beschrieben, wobei die Histidin B10-Reste jeweils dreier Moleküle des Insulinanalogons in einem Hexamer über Wasserstoffbrückenbindungen mit einem Wassermolekül, einem Dihydrogenphosphat-Ion (H₂PO₄⁻), einem Monohydrogenphosphat-Ion (HPO₄²⁻) oder einem Sulfat-Ion (SO₄²⁻) verbunden sind.

Ein weiterer Gegenstand der Erfindung sind Kristalle wie oben beschrieben, wobei die Histidin B10-Reste der Moleküle des Insulinanalogons in einem Hexamer jeweils auf das eigene Dimer zurückgefaltet sind und keine Wasserstoffbrückenbildung der Histidin B10-Reste zu einem Wassermolekül vorhanden ist.

Die erfindungsgemäßen Kristalle enthalten dabei insbesondere ein Insulinanalogon dadurch gekennzeichnet, dass die B-Kette die Sequenz

Phe Val Lys Gln His Leu Cys Gly Ser His Leu Val Glu Ala Leu
Tyr Leu Val Cys Gly Glu Arg Gly Phe Phe Tyr Thr Pro Glu Thr

aufweist (SEQ ID NO 3).

Ein weiterer Gegenstand der Erfindung ist eine pharmazeutische Zubereitung, dadurch gekennzeichnet, dass sie mindestens einen Kristall wie oben beschrieben enthält, wobei bevorzugt ein Hilfsstoff enthalten sein kann, der die Aufnahme des Insulinanalogons ins Blut erleichtert und ganz besonders bevorzugt wobei die pharmazeutische Formulierung eine Insulinaktivität mit schnellem Wirkungseintritt aufweist.

Ein weiterer Gegenstand der Erfindung ist eine pharmazeutische Zubereitung, dadurch gekennzeichnet, dass sie mindestens einen Kristall wie oben beschrieben und einen Hilfsstoff enthält, der bei inhalativen und / oder oralen Formulierungen von Insulin oder Insulinanaloga Anwendung findet.

Ein weiterer Gegenstand der Erfindung ist ein Verfahren zur Herstellung eines Kristalls wie oben beschrieben, bei dem
(a) ein zinkfreies, amorphes Pulver eines Insulinanalogons wie oben beschrieben in einer Konzentration von 15-25 mg/ml in Wasser gelöst wird,
(b) eine Präzipitation mit einem geeigneten Präzipitanten erfolgt, und
(c) die Kristalle isoliert und getrocknet werden;
wobei das Insulinanalogon insbesondere Lys B3, Glu B29-Humaninsulin ist.

Ein weiterer Gegenstand der Erfindung ist ein Verfahren zur Herstellung eines Kristalls wie oben beschrieben, bei dem der Präzipitant ausgewählt wird aus einer Gruppe enthaltend
(a) Ammoniumdihydrogenphosphat,
(b) eine Kombination aus Ammoniumdihydrogenphosphat und Tri-Natriumcitrat; und
(c) eine Kombination aus Ammoniumsulfat und Polyethylenglycol diverser Molekulargewichte;
wobei bevorzugt als Präzipitant Ammoniumdihydrogenphosphat oder Di-Ammoniumhydrogenphosphat bei pH Werten zwischen 3,0 und 8,0, oder Ammoniumdihydrogenphosphat/Di-Ammoniumhydrogenphosphat in Kombination mit Tri-Natriumcitrat bei einem pH-Wert von 5,5 ± 1,5 oder Ammoniumsulfat in Kombination mit PEG diverser Molekulargewichte bei einem pH-Wert von 6,0 ± 1,5 Anwendung findet.

Auch Gegenstand der Erfindung ist die Verwendung eines Kristalls wie oben beschrieben zur Herstellung eines Arzneimittels zur Behandlung von Diabetes der Typen I und / oder II.

Die Erfindung wird anhand der Beispiele näher erläutert, ohne sich darauf zu beschränken.

### Beispiel 1: Herstellung der neuen zinkfreien Hexamer-Kristallform:

Amorphes, zinkfreies Pulver von Lys B3, Glu B29-Humaninsulin wird ungepuffert in aqua dest./HCl bei pH um 2.0 gelöst. Dieses Vorgehen erlaubt, dass sich bei Insulinkonzentrationen um 20 mg/ml eine klare Lösung ausbildet. Die Kristallisation findet nach der Hanging-drop Methode in 24-well Linbro-Platten statt. Als Präzipitant wird das nachfolgende Reagens des "Protein Crystallization Screening Kit" der Firma Hampton Research Inc. verwendet: 1. 0,4 M Ammoniumdihydrogenphosphat, pH 4,2.

### Beispiel 2: Herstellung der neuen zinkfreien Hexamer-Kristallform:

Amorphes, zinkfreies Pulver von Lys B3, Glu B29-Humaninsulin wird ungepuffert in aqua dest./HCl bei pH um 2.0 gelöst. Dieses Vorgehen erlaubt, dass sich bei Insulinkonzentrationen um 20 mg/ml eine klare Lösung ausbildet. Die Kristallisation findet nach der Hanging-drop Methode in 24-well Linbro-Platten statt. Als Präzipitant wird das nachfolgende Reagens des "Protein Crystallization Screening Kit" der Firma Hampton Research Inc. verwendet: 1 M Ammoniumdihydrogenphosphat, 0,1 M Tri-Natriumcitrat, pH 5,6.

### Beispiel 3: Herstellung der neuen zinkfreien Hexamer-Kristallform:

Amorphes, zinkfreies Pulver von Lys B3, Glu B29-Humaninsulin wird ungepuffert in aqua dest./HCI bei pH um 2.0 gelöst. Dieses Vorgehen erlaubt, dass sich bei Insulinkonzentrationen um 20 mg/ml eine klare Lösung ausbildet. Die Kristallisation findet nach der Hanging-drop Methode in 24-well Linbro-Platten statt. Als Präzipitant wird das nachfolgende Reagens des "Protein Crystallization Screening Kit" der Firma Hampton Research Inc. verwendet: 0,2 M Ammoniumsulfat, 20% PEG 3350, pH 6,0.

### Beispiel 4: Röntgenstrukturanalyse

Die gemäß der Beispiele 1-3 erhaltenen Kristalle sind wohl geordnet und ermöglichen eine Röntgenstrukturanalyse, welche für ein Insulin mit Glutamat-B21-Seitenkette ein bisher unbekanntes strukturelles Arrangement der Region um die Histidin-B10-Seitenkette aufzeigt.

Zinkhaltige Humaninsulin-Kristalle des 2Zn-Typus weisen als Raumgruppe R3 (146) und Zellachsen von A=82.5, C=34.0 Å auf. Zinkfreie Kristalle der Verbindung I sind isomorph und weisen typischerweise Zellachsen von A=81.5, C=33.3 Å auf. Die Insulinmoleküle in der zinkhaltigen Kristallstruktur von Humaninsulin und der zinkfreien Kristallstruktur von Lys B3, Glu B29-Humaninsulin im sogenannten T₆- bzw. 2Zn-Typus gefaltet. Im 2Zn-Insulin (T₆) liegen alle 6 Monomere im sogenannten T = tensed Zustand vor. Der N-Terminus der B-Ketten ist ausgesteckt ohne signifikantes Sekundärstrukturmerkmal, es liegt keine □-Helix-Sekundärstuktur wie im alternativen R-Zustand vor. Signifikante Unterschiede in den Kristallstrukturen von Humanainsulin und der Verbindung I ergeben sich im Arrangement der Region um die Histidin-B10-Seitenkette.

Für alle Herstellungsmethoden gemäß der Beispiele 1-3 liegen hochauflösende Einkristall Röntgenstukturanalysen vor, die den zinkfreien Zustand dieser Insulinhexamer-Kristalle beweisen. Als Elementarzellachsen und Kristallsymmetrie wurde bestimmt:
Herstellungsverfahren 1: A=81.52, C=33.31 Å, R3 (Raumgruppe Nr. 146)
Herstellungsverfahren 2: A=81.51, C=33.43 Å, R3 (Raumgruppe Nr. 146)
Herstellungsverfahren 3: A=81.38, C=33.22 Å, R3 (Raumgruppe Nr. 146).

## Patentansprüche

1. Kristalle des Insulinanalogons LysB3, GluB29 Humaninsulin, wobei die Kristalle in der Raumgruppe R3 (Nr. 146) mit den Zellachsen A=81,5 Å ± 1 Å und C= 33,3 Å ± 1 Å vorliegen, wobei die Moleküle des Insulinanalogons in Form von zinkfreien Hexameren, bestehend aus jeweils drei Dimeren, vorliegen.

2. Kristalle gemäß Anspruch 1, wobei die Histidin B10-Reste jeweils dreier Moleküle des Insulinanalogons in einem Hexamer über Wasserstoffbrückenbindungen mit einem Wassermolekül verbunden sind.

3. Kristalle gemäß Anspruch 1, wobei die Histidin B10-Reste jeweils dreier Moleküle des Insulinanalogons in einem Hexamer über Wasserstoffbrückenbindungen mit einem Dihydrogenphosphat-Ion (H₂PO₄⁻) verbunden sind.

4. Kristalle gemäß Anspruch 1, wobei die Histidin B10-Reste jeweils dreier Moleküle des Insulinanalogons in einem Hexamer über Wasserstoffbrückenbindungen mit einem Monohydrogenphosphat-Ion (HPO₄²⁻) verbunden sind.

5. Kristalle gemäß Anspruch 1, wobei die Histidin 810-Reste jeweils dreier Moleküle des Insulinanalogons in einem Hexamer über Wasserstoffbrückenbindungen mit einem Sulfat-Ion (SO₄²⁻) verbunden sind.

6. Kristalle gemäß einem oder mehreren der Ansprüche 1 bis 5, wobei die Histidin B10-Reste der Moleküle des Insulinanalogons in einem Hexamer jeweils auf das eigene Dimer zurückgefaltet sind und keine Wasserstoffbrückenbildung der Histidin B10-Reste zu einem Wassermolekül vorhanden ist.

7. Pharmazeutische Zubereitung, **dadurch gekennzeichnet, dass** sie mindestens einen Kristall nach einem der Ansprüche 1 bis 6 enthält.

8. Pharmazeutische Zubereitung nach Anspruch 7, **dadurch gekennzeichnet, dass** sie ferner einen Hilfsstoff enthält, der die Aufnahme des Insulinanalogons ins Blut erleichtert.

9. Pharmazeutische Zubereitung nach Anspruch 7, **dadurch gekennzeichnet, dass** sie mindestens einen Kristall nach einem der Ansprüche 1 bis 6 und einen Hilfsstoff enthält, der bei inhalativen und / oder oralen Formulierungen von Insulin oder Insulinanaloga Anwendung findet.

10. Verwendung eines Kristalls gemäß einem oder mehreren der Ansprüche 1 bis 6 zur Herstellung einer pharmazeutischen Zubereitung, welche eine Insulinaktivität mit schnellem Wirkungseintritt aufweist.

11. Verfahren zur Herstellung eines Kristalls gemäß einem der Ansprüche 1 bis 6, bei dem
(a) ein zinkfreies, amorphes Pulver des Insulinanalogons gemäß der Ansprüche 1 bis 10 in einer Konzentration von 15-25 mg/ml in Wasser gelöst wird,
(b) eine Präzipitation mit einem geeigneten Präzipitanten erfolgt, und
(c) die Kristalle isoliert und getrocknet werden.

12. Verfahren gemäß Anspruch 11, bei dem der Präzipitant ausgewählt wird aus einer Gruppe enthaltend
(a) Ammoniumdihydrogenphosphat,
(b) eine Kombination aus Ammoniumdihydrogenphosphat und Tri-Natriumcitrat; und
(c) eine Kombination aus Ammoniumsulfat und Polyethylenglycol diverser Molekulargewichte.

13. Verfahren zur Herstellung der Kristalle gemäß einem oder mehreren der Ansprüche 1, 3, 4 und 6 nach einem Verfahren gemäß Anspruch 12, wobei als Präzipitant Ammoniumdihydrogenphosphat oder Di-Ammoniumhydrogenphosphat bei pH Werten zwischen 3,0 und 8,0 Anwendung findet.

14. Verfahren zur Herstellung der Kristalle gemäß einem oder mehreren der Ansprüche 1 bis 6 nach einem Verfahren gemäß Anspruch 12, wobei als Präzipitant entweder Ammoniumdihydrogenphosphat/Di-Ammoniumhydrogenphosphat in Kombination mit Tri-Natriumcitrat bei einem pH-Wert von 5,5 ± 1,5 oder Ammoniumsulfat in Kombination mit PEG diverser Molekulargewichte bei einem pH-Wert von 6,0 ± 1,5 Anwendung findet.

15. Verwendung eines Kristalls nach einem der Ansprüche 1-6 zur Herstellung eines Arzneimittels zur Behandlung von Diabetes der Typen I und / oder II.

## Claims

1. A crystal of the insulin analog LysB3,GluB29-human insulin, the crystal being present in the space group R3 (No. 146) with the cell axes A = 81.5 Å ± 1Å and C = 33.3 Å ± 1 Å, where the molecules of the insulin analog are present in the form of the zinc-free hexamers consisting of in each case three dimers.

2. The crystal as claimed in claim 1, where the histidine B10 residues of in each case three molecules of the insulin analog in a hexamer are bonded to a water molecule via hydrogen bonds.

3. The crystal as claimed in claim 1, where the histidine B10 residues of in each case three molecules of the insulin analog in a hexamer are bonded to a dihydrogenphosphate ion (H₂PO₄⁻) via hydrogen bonds.

4. The crystal as claimed in claim 1, where the histidine B10 residues of in each case three molecules of the insulin analog in a hexamer are bonded to a monohydrogenphosphate ion (HPO₄²⁻) via hydrogen bonds.

5. The crystal as claimed in claim 1, where the histidine B10 residues of in each case three molecules of the insulin analog in a hexamer are bonded to a sulfate ion (SO₄²⁻) via hydrogen bonds.

6. The crystal as claimed in one or more of claims 1 to 5, where the histidine B10 residues of the molecules of the insulin analog in a hexamer are in each case folded back onto their own dimer and no hydrogen bond formation of the histidine B10 residues to a water molecule is present.

7. A pharmaceutical preparation which comprises at least one crystal as claimed in one of claims 1 to 6.

8. The pharmaceutical preparation as claimed in claim 7, which further contains an excipient which facilitates the absorption of the insulin analog into the blood.

9. The pharmaceutical preparation as claimed in claim 7, which contains at least one crystal as claimed in one of claims 1 to 6 and an excipient which is used in inhalative and/or oral formulations of insulin or insulin analogs.

10. The use of a crystal as claimed in one or more of claims 1 to 6 in the manufacture of a pharmaceutical preparation which has an insulin activity having a rapid onset of action.

11. A process for the preparation of a crystal as claimed in one of claims 1 to 11, in which
(a) a zinc-free, amorphous powder of an insulin analog as claimed in claims 1 to 10 is dissolved in water in a concentration of 15-25 mg/ml,
(b) precipitation using a suitable precipitant is carried out, and
(c) the crystals are isolated and dried.

12. The process as claimed in claim 11, in which the precipitant is selected from a group comprising
(a) ammonium dihydrogenphosphate,
(b) a combination of ammonium dihydrogenphosphate and trisodium citrate; and
(c) a combination of ammonium sulfate and polyethylene glycol of various molecular weights.

13. A process for the preparation of the crystal as claimed in one or more of claims 1, 3, 4 and 6 by a process as claimed in claim 12, where the precipitant used is ammonium dihydrogenphosphate or diammonium hydrogenphosphate at pHs between 3.0 and 8.0.

14. A process for the preparation of the crystals as claimed in one or more of claims 1 to 6 by a process as claimed in claim 12, where the precipitant used is either ammonium dihydrogenphosphate/diammonium hydrogenphosphate in combination with trisodium citrate at a pH of 5.5 ± 1.5 or ammonium sulfate in combination with PEG of various molecular weights at a pH of 6.0 ± 1.5.

15. The use of a crystal as claimed in one of claims 1 - 6 in the manufacture of a pharmaceutical for the treatment of diabetes of types I and/or II.

## Revendications

1. Cristaux de l'analogue d'insuline LysB3,GluB29-insuline humaine, où les cristaux se trouvent dans le groupe spatial R3 (n° 146) avec les axes cellulaires A = 81,5 Å ± 1Å et C = 33, 3 Å ± 1 Å, les molécules de l'analogue d'insuline se trouvant sous forme d'hexamères exempts de zinc, constitués par à chaque fois trois dimères.

2. Cristaux selon la revendication 1, où les restes d'histidine B10 à chaque fois de trois molécules de l'analogue d'insuline sont reliés dans un hexamère via des liaisons pont hydrogène avec une molécule d'eau.

3. Cristaux selon la revendication 1, où les restes d'histidine B10 à chaque fois de trois molécules de l'analogue d'insuline sont reliés dans un hexamère via des liaisons pont hydrogène avec un ion dihydrogénophosphate (H₂PO₄⁻).

4. Cristaux selon la revendication 1, où les restes d'histidine B10 à chaque fois de trois molécules de l'analogue d'insuline sont reliés dans un hexamère via des liaisons pont hydrogène avec un ion monohydrogénophosphate (HPO₄²⁻).

5. Cristaux selon la revendication 1, où les restes d'histidine B10 à chaque fois de trois molécules de l'analogue d'insuline sont reliés dans un hexamère via des liaisons pont hydrogène avec un ion sulfate (SO₄²⁻).

6. Cristaux selon l'une ou plusieurs des revendications 1 à 5, où les radicaux d'histidine B10 des molécules de l'analogue d'insuline dans un hexamère sont à chaque fois repliés sur leur propre dimère et où il n'existe pas de formation de pont hydrogène des restes d'histidine B10 avec une molécule d'eau.

7. Préparation pharmaceutique, **caractérisée en ce qu'**elle contient au moins un cristal selon l'une quelconque des revendications 1 à 6.

8. Préparation pharmaceutique selon la revendication 7, **caractérisée en ce qu'**elle contient en outre un adjuvant qui facilite l'absorption de l'analogue d'insuline dans le sang.

9. Préparation pharmaceutique selon la revendication 7, **caractérisée en ce qu'**elle contient au moins un cristal selon l'une quelconque des revendications 1 à 6 et un adjuvant qui est utilisé dans les formulations par inhalation et/ou orales d'insuline ou d'analogues d'insuline.

10. Utilisation d'un cristal selon l'une ou plusieurs des revendications 1 à 6 pour la confection d'une préparation pharmaceutique qui présente une activité d'insuline avec un début d'activité rapide.

11. Procédé pour la préparation d'un cristal selon l'une quelconque des revendications 1 à 6, dans lequel
(a) une poudre amorphe, exempte de zinc, de l'analogue d'insuline selon les revendications 1 à 10 est dissoute dans l'eau en une concentration de 15-25 mg/ml,
(b) une précipitation est réalisée avec des agents de précipitation appropriés et
(c) les cristaux sont isolés et séchés.

12. Procédé selon la revendication 11, dans lequel l'agent de précipitation est choisi dans un groupe contenant
(a) le dihydrogénophosphate d'ammonium,
(b) une combinaison de dihydrogénophosphate d'ammonium et de citrate trisodique ; et
(c) une combinaison de sulfate d'ammonium et des polyéthylèneglycols de divers poids moléculaires.

13. Procédé pour la préparation des cristaux selon l'une ou plusieurs des revendications 1, 3, 4 et 6 selon un procédé selon la revendication 12, où on utilise comme agent de précipitation du dihydrogénophosphate d'ammonium ou de l'hydrogénophosphate diammonique à des pH entre 3,0 et 8,0.

14. Procédé pour la préparation des cristaux selon l'une ou plusieurs des revendications 1 à 6 selon un procédé selon la revendication 12, où on utilise comme agent de précipitation soit du dihydrogénophosphate d'ammonium/hydrogénophosphate diammonique en combinaison avec du citrate trisodique à un pH de 5,5 ± 1,5, soit du sulfate d'ammonium en combinaison avec des PEG de divers poids moléculaires à un pH de 6,0 ± 1,5.

15. Utilisation d'un cristal selon l'une quelconque des revendications 1 à 6 pour la préparation d'un médicament destiné au traitement du diabète de type I et/ou II.
